# EUROPEAN PATENT APPLICATION

(11) **EP 1 217 312 A2**
(43) Date of publication of application: **26.06.2002**
(21) Application number: 01130301.3
(22) Date of filing: 19.12.2001
(51) Int. Cl.: F24F 3/16, F24F 1/00, A61L 9/16, A61L 2/04, A61L 2/20, A61L 9/015

(54) **Air conditioner and method for inhibiting mold growth in said air conditioner**

(30) Priority: 20.12.2000 JP 2000386643
(71) Applicant: MATSUSHITA ELECTRIC INDUSTRIAL CO., LTD., Kadoma-shi, Osaka 571-8501 (JP)
(72) Inventor: Sato, Shigehiro, Kusatsu-shi, Shiga 525-0045 (JP); Nakao, Keiji, Otsu-shi, Shiga 520-0837 (JP); Nishihara, Yoshikazu, Kouga-gun, Shiga 529-1851 (JP); Iseki, Takayuki, Otsu-shi, Shiga 520-2144 (JP); Arashima, Hiroshi, Kusatsu-shi, Shiga 525-0058 (JP)
(74) Representative: Grünecker, Kinkeldey, Stockmair & Schwanhäusser Anwaltssozietät

(57) **Abstract**

It is an object of the present invention to provide an air conditioner capable of inhibiting mold growth in a body thereof, and a method for inhibiting mold growth in the air conditioner. According to the air conditioner of the invention, at least either one of a heating means and a mold-preventing means is provided in the body, and the interior of the body is brought under a predetermined condition, thereby inhibiting mold growth at least in a blowing means of the air conditioner. According to the method for inhibiting mold growth in the air conditioner, the interior of the body is heated under a specific condition or supplied with a specific fungicide, thereby inhibiting the mold growth in the air conditioner to an extent of practically causing no problem.

## Description

### TECHNICAL FIELD

The present invention relates to an air conditioner having a function for inhibiting mold growth, and a method for inhibiting mold growth in the air conditioner.

### BACKGROUND TECHNIQUE

A body of an air conditioner is provided with an air-intake port, a heat exchanger, a blower and an air-blowoff port, as air-blowing means. An air filter is provided in the air-intake port. According to the air conditioner having such air-blowing means, since moisture condensation is prone to be generated in the heat exchanger, humidity in the body becomes high, and mold is prone to grow in the air-blowing means or the air filter. Thereupon, various attempts have been made to inhibit mold from growing in the air conditioner.

In general, a fungicide comprising a compound of silver or copper is applied to the heat exchanger, the air-blower or the air filter where the mold is prone to be generated, thereby inhibiting mold from growing. However, if the air conditioner is used for a long term, dust settles on a component on which the fungicide was applied, effect of the fungicide can not be obtained sufficiently, and mold is prone to grow on the accumulated dust.

To solve such a problem, it has been considered to use a fungicide which directly acts on mold in a vapor phase inside the body. According to such fungicide, however, fungicide components must be carried on a porous medium and must be disposed inside the air conditioner, and it is difficult to reduce the fungicide in size and to elongate the life thereof.

There is a proposed air conditioner in which a fungicide function is provided in the air conditioner to sterilize mold or germ in a vapor phase inside a body of the air conditioner. An ultraviolet lamp and an electrical discharge mechanism for generating ozone and negative ion are used as fungicide mechanism. For example, Japanese Patent Application Laid-open No. 9-119657 discloses a refrigerating and air conditioner having a bacteria growth preventing mechanism which generates negative ion while lowering concentration of ozone to sterilize bacteria. This bacteria propagation preventing mechanism comprises an ion chamber for ionizing gas in air, and an ozone decomposing chamber for removing ozone included in the ionized gas, but its structure is complicated. Further, this mechanism requires to be of a certain size and thus, there is a problem that it is difficult to incorporate this mechanism in the body. Even if the mechanism could be incorporated in the body, there arises a problem that the system can not be reduced in size Furthermore, since ozone is harmful to human, it is preferable not to use such ozone. There is also a problem that an air conditioner using an ultraviolet lamp as a fungicide mechanism can not be reduced in size if the ultraviolet lamp is provided in the body.

Thereupon, there is a proposed air conditioner in which humidity in the air conditioner is lowered to inhibit mold from growing instead of providing the above-described fungicide function. For example, there is a proposed method in which drying operation is carried out to lower the humidity in the body. According to this method, however, when the drying operation is carried out, air-blowing operation alone is carried out, so that the humidity in the air conditioner is not lowered so much, and sufficient mold-preventing effect can not be obtained. Japanese Patent Publication No. 7-69068 discloses an air conditioner in which cooling operation is carried out for a given time and then, heating operation is carried out for a given time and at that time, short-circuit operation is carried out, blown air current is sucked in the body, and an interior of the body is dried. However, even if the heating operation is carried out to lower the humidity in the body, if the frequency of the heating operation is low, sufficient mold-preventing effect can not be obtained, and growth of mold starts soon in some cases.

Japanese Unexamined Patent Application No. 5-141692 discloses an air conditioner in which a short-circuit flow-path is provided in the air conditioner, high temperature air is short-circuited to sterilize the interior of the air conditioner. In order to sterilize, however, the interior of the air conditioner must be maintained at a high temperature of 80 °C or more, and it is necessary that resin components constituting the air conditioner be changed to another material capable of withstanding such high temperature.

It is an object of the present invention to solve the above problems, and to provide an air conditioner capable of inhibiting growth of mold in a body of the air conditioner, and to provide a mold-growth inhibiting method in the air conditioner.

### DISCLOSURE OF THE INVENTION

An air conditioner of the present invention is characterized in that at least one of a heating means and a mold inhibiting means is provided in a body of the air conditioner, an interior of the body is exposed to a specific condition, thereby inhibiting mold growth at least in an air blowing means.

According to the present invention, it is possible to satisfactorily inhibit mold growth in the air conditioner with a simplified configuration of the conditioner, and to reduce the size of the conditioner.

A mold-growth inhibiting method for an air conditioner of the present invention is characterized in that the interior of a body of the conditioner is heated under a specific condition or supplied with a specific fungicide.

According to the present invention, it is possible to inhibit the growth of mold in the air conditioner to such an extent of causing practically no problem.

### BRIEF DESCRIPTION OF THE DRAWINGS

Fig.1 is a sectional view of an indoor unit of an air conditioner according to an embodiment of the present invention;
Fig.2 is a sectional view of the indoor unit of the air conditioner having a heater as a heating means; and
Fig.3 is a sectional view of the indoor unit of the air conditioner having a Peltier device as a heating means.

### BEST MODE FOR CARRYING OUT THE INVENTION

An air conditioner of the present invention generally means a system in which humidity in a body of the system is increased by cooling or dehumidification operation. Examples of such an air conditioner are a wall-mounted separate type air conditioner, a ceiling built-in type separate type air conditioner, an integral type window air conditioner, a vehicle air conditioner and the like.

The following explanation is based on the wall-mounted separate type air conditioner, and especially an indoor unit of the air conditioner having a problem of mold will be explained.

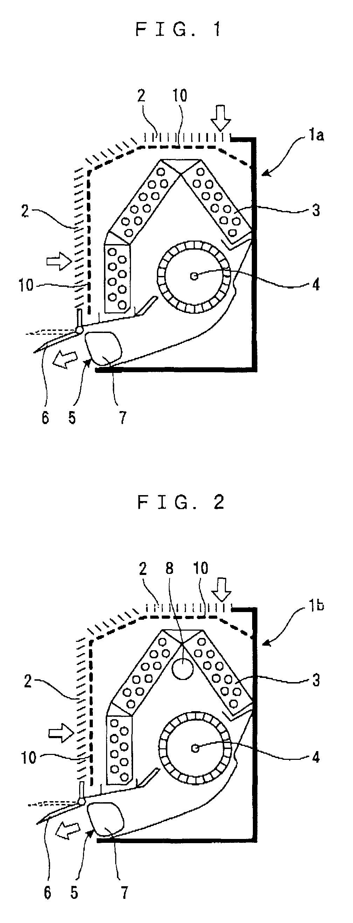

Fig.1 shows the air conditioner of the present invention.

In an indoor unit 1a as a body, an air-intake port 2, a heat exchanger 3, a blower 4 and an air-blowoff port 5 are provided as air-blowing means. The air-blowoff port 5 is provided with an air-blowoff blade 6 capable of opening and closing, and laterally-changing blades 7 for adjusting the direction of wind. A reference number 10 represents an air filter.

In the air conditioner having the above-described structure, if the blower 4 rotates, outside air is drawn from the air-intake port 2. At that time, spore of mold floating in the air is also drawn into the indoor unit 1a together with air, and adhered to a surface of a component constituting the indoor unit 1a by static electricity or together with contamination such as oil mist.

Mold which is prone to be grown in the air conditioner is hygrophilous mold widely existing in a normal indoor, and is Cladosporium, Alternaria, Aspergillus or the like for example. A size of the spore is usually 100µm or less. Growing speed of mold is largely varied depending upon temperature or humidity and usually, the growing speed becomes fast when the temperature is in a range of 20 °C to 30 °C and humidity is as high as 70 %RH or higher.

In the air conditioner, if cooling operation or dehumidification operation is carried out, moisture condensation is prone to be generated in the heat exchanger 3, and the humidity in the indoor unit 1a becomes higher. More specifically, during the cooling operation or dehumidification operation, the humidity in the indoor unit is about 100 %RH, and even when the system is stopped, the humidity is maintained at 90 %RH or higher for a long time.

Therefore, if the cooling operation or dehumidification operation is carried out in a state in which spore of mold is adhered inside the indoor unit 1a, germination of spore of mold, growth of hypha, and reproduction of spore take place, and the inside of the indoor unit 1a becomes polluted with mold. Portions which are to be seriously polluted are portions constituting air-paths such as the heat exchanger 3, the blower 4, the air-blowoff blade 6 and the air filter 7. Especially when mold grows on a surface of blades of the blower 4, the growth is repeated between the blades, and a gap between the blades of the blower 4 is blocked with mold, and no wind comes out from the air-blowoff port 5 in some cases.

Thereupon, in the present invention, at least one of a heating means for inhibiting mold growth or a mold-preventing means is provided in the air conditioner, the growth of mold in the air conditioner is inhibited to such an extent of causing practically no problem, by using a mold-growth inhibiting method. In the present invention, at least one of the heating means or mold-preventing means is provided to inhibit the mold growth under specific condition when an interior of the indoor unit was brought into a condition suitable for breeding mold. More specifically, it is unnecessary to carry out the present invention when heating operation is carried out, and it is necessary to carry out the present invention when cooling operation or the like is carried out.

First, an air conditioner having the heating means will be explained.

In the air conditioner of the invention, the indoor unit has the heating means, and it is necessary to carry out the heating process at a temperature of 36 °C or higher for 10 minutes or longer at least once a day.

If the heating temperature becomes lower than 36 °C, sufficient mold-growth inhibiting effect can not be obtained. The heating temperature may be 36 °C or higher, but if the temperature exceeds 80 °C, the mold-growth inhibiting effect is almost the same. As the heating temperature is increased, components are more prone to be deteriorated. Therefore, in the present invention, it is preferable that the heating temperature is in a range of 40 to 80 °C and more preferably, in a range of 40 to 60 °C.

If the heating time is shorter than 10 minutes, sufficient mold-growth inhibiting effect can not be obtained. The heating time may be 10 minutes or longer, but if the time exceeds 60 minutes, the effect is almost the same. If the heating time is excessively long, the components are more prone to be deteriorated, and energy is consumed uselessly. Therefore, in the present invention, it is preferable that the heating time is in a range of 10 to 60 minutes and more preferably, in a range of 10 to 30 minutes.

It is necessary to carry out the above-described heating process at least once a day. If the above-described heating process is not carried out at least once a day, sufficient mold-growth inhibiting effect can not be obtained. Although it is necessary to carry out the heating process every day, if the heating process is excessively carried out, which may cause deterioration of components and cost up. Therefore, in the present invention, it is preferable to carry out the heating process up to four times a day.

Such a heating process can only inhibits growth of mold but can not deaden the mold, but if the heating process is repeatedly carried out at least once every day, it is possible to inhibit growth of hypha, to suppress adhesion of spore, and to inhibit mold growth in the air conditioner finally. Since the interior temperature in the indoor unit at the time of heating operation is about 60 °C at the highest, a conventionally used components can be used as they are as resin components constituting the indoor unit.

The heating method is not limited especially, and a heater for carrying out usual heating operation provided in an air conditioner may be used for example. Examples of types of the heater are heat-pump type, coolant heating type or hot water type. If such a previously incorporated heater is used, since it is unnecessary to increase the number of components, miniaturization of the system is not hindered. When the heating process is carried out by using the heating operation which is previously incorporated, the heating process is carried out under a condition that the cooling operation is once stopped one time or more a day in a cooling operation season. In a heating season, it is unnecessary to carry out heating-mold preventing process. At the time of heating operation, since a temperature in the air conditioner is 36 °C or higher and humidity is 60 % or less, mold does not grow and mold-preventing operation is unnecessary. If the operation state is abruptly switched from the cooling operation to the heating operation, water adhering to the heat exchanger at the time of cooling operation evaporates abruptly, which increases humidity in the room and which grates. Therefore, the water on the heat exchanger should be discharged into drain by blowing operation. When the cooling operation is carried out, outside temperature is high. If the heating operation is carried out in this state, load on a compressor is increased and thus, the heating operation should be limited to minimum time required for the heating mold-preventing process.

When the heating process is carried out by the heating operation as described above, it is preferable that the air conditioner is provided with control means for controlling the heating mold-preventing process, and if instructions for executing the heating mold-preventing process are given, the heating process is carried out at a set operation-start time for a predetermined time at a predetermined temperature. It is also preferable that the control means is programmed such that after the heating mold-preventing process was completed, the operation state is automatically returned to the original cooling operation. More specifically, it is preferable that a button for heating mold-preventing process is provided on an input setting device provided in the air conditioner, and if a person who uses the air conditioner sets time for executing the heating mold-preventing process by means of the button, the heating mold-preventing process is executed at a predetermined time utilizing a timer incorporated in the input setting device. If such control means is provided, a user can specify the starting time of the heating mold-preventing operation by himself or herself and thus, it is possible to reliably carry out the heating mold-preventing process.

Heating means may newly be provided.

For example, a heater 8 may be disposed in an indoor unit 1b to carry out the heating process as shown in Fig.2. It is preferable to dispose the heater 8 in such a place that draft in the indoor unit 1b is not hindered as small as possible.

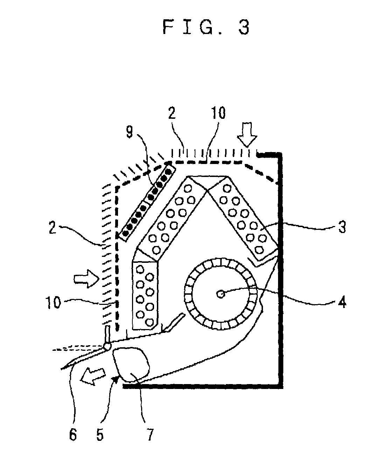

Even if a Peltier device 9 is provided in a indoor unit 1c instead of the heater 5 as shown in Fig.3, the same effect can be obtained. Although the Peltier devices 9 are disposed side-by-side on the outer surface of the heat exchanger 3, if the Peltier devices 9 are disposed on places where draft in the indoor unit 1c is not hindered as small as possible, the places are not limited especially.

In the air conditioner of the present invention, since it is possible to provide the heating means with simple technique and without increasing the number of components so much, the size of the air conditioner can be reduced.

The heating means may be used singly, or a plurality of heating means may be combined and used.

When the heating process is carried out, it is preferable to do so in a state in which the air-blowoff port 5 of the indoor unit (1a, 1b or 1c) is closed. For example, if the heating process is carried out while the air-blowoff blade 6 provided in the opening of the air-blowoff port 5 is closed, the interior of the indoor unit is entirely heated, which is effective to inhibit the mold growth. Further, since warm air is not discharged from the air conditioner, it is possible to reduce the annoyance of the user when the mold-preventing process is carried out.

When the heating process is carried out, it is preferable that the volume of air sent by the blower fan is smaller than the volume of air when the normal cooling operation is carried out, because a temperature in the indoor unit can be heated to 36 °C or higher swiftly.

In the air conditioner having such heating means, it is more preferable that the control means carries out the above action. For example, if the mold-preventing process by the heating process is instructed, it is more preferable to provide control means which controls such that the air-blowoff ports 5 of the indoor units 1a to 1c are closed, and the volume of air of the blower fan is smaller than that of the normal cooling operation is carried out.

It is preferable that the fungicide comprising compound such as conventionally used silver or copper is applied to the constituent components of the blower means where mold is prove to be generated, because mold is less prone to be generated on the components more effectively and mold growth in a vapor phase can effectively be inhibited.

Next, the air conditioner having the mold-preventing means will be explained.

It is necessary to dispose, in the indoor unit, means for supplying gas including isothiocyanate, and the components constituting the blowing means is exposed to atmosphere having 0.1 ppm or higher isothiocyanate in the vapor phase at least once a day for 10 minutes or longer.

In the present invention, it is necessary to use the isothiocyanate as the mold-preventing means. The isothiocyanate is liquid under a normal temperature, but has high vapor pressure under the normal temperature, and some isothiocyanate has vapor pressure of about 1000 ppm in its saturation. The isothiocyanate inhibits breathing of mold, and is material having high mold-preventing effect. If gas having 0.1 ppm or more of the isothiocyanate is supplied into the indoor unit at least once a day for 10 minutes or longer, it is possible to prevent reproduction of mold spore in the indoor unit and to inhibit the mold growth.

If the concentration of the isothiocyanate in the vapor phase is lower than 0.1 ppm, sufficient mold-growth inhibiting effect can not be obtained. If the concentration of the isothiocyanate is 0.1 ppm or more, it is possible to inhibit the mold growth effectively, but if the concentration of the isothiocyanate is excessively high, smell of the isothiocyanate is adversely transmitted outside an outdoor unit. Therefore, if the air conditioner is used while human is living, it is preferable to adjust the concentration of the isothiocyanate in the indoor unit near the air-blowing exit to 10 ppm or lower.

The isothiocyanate inhibits breathing of the mold, and in order to inhibit the mold growth and to obtain the mold-preventing effect, it is considered that certain time period is required. Therefore, if the isothiocyanate is not supplied once a day, sufficient mold-preventing effect can not be obtained. Similarly, if the supplying time is shorter than 10 minutes, sufficient mold-preventing effect can not be obtained. The exposing time of the air conditioner with the isothiocyanate can be changed depending upon the concentration of the isothiocyanate, and if the concentration is increased as high as possible, it is possible to carry out the mold-preventing process in a short time within one hour.

Examples of the isothiocyanate are allyl isothiocyanate, methyl isothiocyanate, phenyl isothiocyanate, 4-methylthio-3-butenyl isothiocyanate. In the present invention, the allyl isothiocyanate is optimal material. The allyl isothiocyanate has high mold-preventing effect, has smell component of horse-radish, and high safety and thus, it can be used preferably.

The supply means for supplying the gas including isothiocyanate into the indoor unit is not especially limited only if the supply means periodically supplies the gas including isothiocyanate into the indoor unit. For example, a bag is put on a portion of the indoor unit from its air-sucking port to its air-blowing exit, isothiocyanate having a predetermined concentration is supplied into the bag, and the gas including the isothiocyanate can be supplied to the indoor unit periodically.

In the present invention, isothiocyanate and resin are mixed to lower the vapor pressure, and the mixture may be used as the mold-preventing means. Even if the resin mixture having low vapor pressure is provided in the indoor unit and the concentration of the isothiocyanate in the vapor phase is set to 0.1 ppm or higher, it is possible to prevent the reproduction of mold spore in the blowing path, the heat exchanger, the fan and the filter, to prevent breeding of the mold and to inhibit mold growth in the same manner as described above. Although it is difficult to actually measure the vapor pressure of the resin component, it is possible to obtain the vapor pressure by measuring the discharge speed of the isothiocyanate. For example, if the atmosphere in the indoor unit is maintained at 30 °C and 95 % HR or lower and the discharge speed of the isothiocyanate is controlled to 25 mold growth/day, excellent mold-preventing effect can be obtained. If this control is not carried out, the discharge speed is 1 g/day or faster.

The resin component including the isothiocyanate can preferably be used because the concentration of the isothiocyanate can easily be controlled. Since the resin component does not require constant size, this does not hinder miniaturization of the system.

Urethane resin, acrylic resin, rosin ester can preferable used as resin to be mixed with isothiocyanate.

In the present invention, paint including isothiocyanate and having gradually-releasing characteristic can also be used as another mold-preventing means. If such paint is applied to at least one of constituent components of the blowing means and the concentration of the isothiocyanate in the vapor phase of the body is set to 0.1 ppm or higher, effect for inhibiting the mold growth on the component can be obtained, and which is more preferable.

The "gradually-releasing characteristic" of isothiocyanate in the present invention means a state in which the concentration of the isothiocyanate in the vapor phase is 1 ppm to 500 ppm for one month or longer.

Paint in which micro capsules of isothiocyanate are dispersed in water solution mainly comprising urethane resin or urethane-acrylic resin can preferably be used as paint including isothiocyanate and having gradually-releasing characteristic. Such paint has high adhering strength with respect to polystyrene which is widely used for a component constituting an inside portion of the indoor unit and thus, it is used preferably. Since the micro capsules of isothiocyanate are surrounded by resin, isothiocyanate is less prone to be vaporized into air through the resin, and the gradually-releasing characteristic is realized In the temperature environment of the indoor unit of the air conditioner, gradually-releasing characteristic of isothiocyanate can be maintained for about three months and thus, it is effective to apply immediately before the cooling operation season. It is more preferable to apply paint having gradually-releasing characteristic because it is easy to uniform the concentration of the isothiocyanate.

The applying method of paint having isothiocyanate and gradually-releasing characteristic is not especially limited, and a common method can be used. For example, it is preferable that the paint is put into an atomizer and the paint is sprayed to components constituting the blowing means such as the blower and air-blowoff blade of the indoor unit, because even those components deep in the indoor unit can easily be processed. A method in which the paint is put into a cylindrical container, an exit of the paint is provided with an applying apparatus, and the paint is applied to the surface of the component such that the paint seeps through mesh of fiber of the applying apparatus is preferable because the paint can be applied to wider range.

In the present invention, if both the heating means and the mold-preventing means are provided, more excellent mold-growth inhibiting effect can be obtained.

For example, heating means such as the heater 8 or the Peltier device 9 is provided, and paint having isothiocyanate and gradually-releasing characteristic is applied to surfaces of components constituting wind-path such as the blower or heat exchanger.

With this structure, if an inner temperature of the indoor unit is increased and becomes 36 °C by the heating means, isothiocyanate component of the paint applied to the surfaces of components constituting the wind-path becomes prone to be charged into vapor phase. At that time, it is more effective if the concentration of isothiocyanate becomes 0.1 ppm or higher.

Alternatively, when the interior of the indoor unit is heated, gas including isothiocyanate may be supplied into the indoor unit. With this structure also, excellent mold-growth inhibiting effect can be obtained.

Instead of applying isothiocyanate having specific concentration to the surface of the component constituting the wind-path, if the gas including isothiocyanate is supplied into the indoor unit at the same time when the interior of the indoor unit is heated, two growth inhibiting factors, i.e., growth inhibiting factor by means of heating and breathing inhibiting factor by isothiocyanate can be applied to the mold. This is more preferable.

When the heating operation and the isothiocyanate are used together, if the mold-preventing operation time is set by an input setting device, a user can recognize the mold-preventing operation and this is preferable. For example, if time reaches the mold-preventing operation time, the cooling operation is first stopped, and the operation state is switched to the heating mold-preventing operation. At that time, gas including isothiocyanate is simultaneously charged into the indoor unit. As a method for charging the gas including isothiocyanate into the indoor unit, it is possible to use a method in which a container accommodating the isothiocyanate liquid is opened in the indoor unit, a method in which liquid isothiocyanate is injected into the indoor unit, and the like. When the container accommodating the isothiocyanate liquid is opened in the indoor unit, it is preferable if the liquid isothiocyanate is allowed to be carried on expanded body and put in a container, because it is possible to inhibit leakage of the liquid to outside, and the concentration of the isothiocyanate in the indoor unit can easily be controlled. The method for injecting the liquid isothiocyanate into the indoor unit is not especially limited. For example, a method in which a head is vibrated when voltage is applied utilizing a piezoelectric device to inject liquid is preferable because the dispersing amount can finely be controlled.

Concrete embodiments will be shown below.

In the following embodiments and comparative examples, various physical properties were measured by the following method:
(1) AITC concentration (ppm): this was measured using a handy gas chromatography (model No.Voyager made by Perkin Elmer).
(2) Hypha growing speed (µm/day) : hypha growing speed of Alternaria sp. S-78 was measured and judged by microscopic observation using a water-proof mold sensor (made by Environmental biology research institute). The Alternaria sp. S-78 hygrophilous mold, and typical example thereof are Alternaria or Cladosporium generated in air conditioner.

### Embodiments 1 to 3

Using an air conditioner having the same structure as that shown in Fig.1, cooling operation of 26 °C was continuously carried out 12 hours a day. A room temperature was set to 27 °C and humidity was set to 60 %RH.

When the cooling operation was stopped, allyl isothiocyanate ("AITC", hereinafter) of predetermined concentration was supplied into the indoor unit three hours every day. The AITC gas was supplied in such a manner that a drawing grill and an air-blowoff grill of the air conditioner were covered with a bag from its front surface side, and the AITC gas of concentration (volume concentration) shown in Table 1 was supplied into the bag together with air of 0.1M³.

The above operation was repeated for seven days, and the mold-growth state in the indoor unit after seven days was measured.

The obtained mold-growing speed and the like are shown in Table 1.

**Table 1**

| | AITC concentration (ppm) | AITC supply frequency | Hypha growing speed (µm/day) |
|---|---|---|---|
| Embodiment 1 | 40 | Once a day | 320 |
| Embodiment 2 | 50 | Once a day | 200 |
| Embodiment 3 | 60 | Once a day | 100 |
| Comparative example 1 | -- | -- | 420 |
| Comparative example 2 | 0.06 | Once a day | 410 |
| Comparative example 3 | 1.0 | Once a week | 430 |
| Comparative example 4 | 1.0 | Once for two days | 400 |

As shown in embodiments 1 to 3, as the concentration of AITC gas supplied into the indoor unit was higher, more excellent mold-growth inhibiting effect was obtained.

### Comparative Example 1

AITC gas was not supplied into the indoor unit. Other conditions were the same as those of the embodiment 1, and the mold-growth state after seven days was measured.

The obtained measurement result is shown in Table 1.

### Comparative Example 2

The concentration of AITC gas supplied into the indoor unit was set lower than a range of the present invention. Other conditions were the same as those of the embodiment 1, and the mold-growth state after seven days was measured.

The obtained measurement result is shown in Table 1.

### Comparative Example 3

The AITC gas was supplied into the indoor unit once a week. Other conditions were the same as those of the embodiment 1, and the mold-growth state after two weeks was measured.

The obtained measurement result is shown in Table 1.

### Comparative Example 4

The AITC gas was supplied into the indoor unit once for two days. Other conditions were the same as those of the embodiment 1, and the mold-growth state after seven days was measured.

The obtained measurement result is shown in Table 1.

In the comparative example 1, since AITC gas was not supplied, the mold-growth inhibiting effect was not obtained. In the comparative example 2, although the AITC gas was supplied, since the concentration of the gas was too low, sufficient mold-growth inhibiting effect was not obtained. In the comparative example 3, although the concentration of the AITC gas was high, since the gas was supplied only once a week, the mold-growing speed was almost the same as a case in which no AITC gas was supplied, and no effect was obtained. In the comparative example 4, since the supplying frequency of the AITC gas was out of the range of the invention, sufficient mold-growth inhibiting effect was not obtained.

### Embodiments 4 to 7

AITC paint having gradually-releasing characteristic was applied onto surfaces of components shown in Table 2 with methods shown in Table 2.

The AITC paint having gradually-releasing characteristic was prepared by mixing AITC micro capsules (Daiichi Kougyou Seiyaku, Wasaoro Capsule SR) and water-soluble urethane resin (Daiichi Kougyou Seiyaku, Super flex) in the proportion of 1:1. The concentration of AITC was 5%. Here, water-soluble emulsion of 25% solid was used as the super flex.

This paint was applied on the surfaces of predetermined components until film thickness reached 20µm. The paint was applied in the following two methods: a method in which AITC paint solution was put into an atomizer and sprayed like the atomizer, and a method in which AITC paint solution was put into a cylindrical container, and the paint solution was applied to an exit of the container by a paint cloth apparatus having a sponge surface covered with cloth.

The concentration of the AITC in the vapor phase in the indoor unit after seven days was measured. The growing speed of hypha was measured like the previous embodiments.

The obtained mold-growing speed and the like are shown in Table 2. A comparative example 1 is shown so that the results can be compared and considered.

Paint used in the embodiments 4 to 7 was paint in which AITC is held in urethane resin film and had gradually-releasing characteristic constructed such that the paint was gradually evaporated from the surface of the component with time. Further, since the concentration of AITC was equal to or higher than 0.1 ppm which is within the range of the present invention, any of the embodiments could obtain excellent growing inhibiting effect of hypha.

**Table 2**

| | Component to which paint is applied | AITC application method | AITC concentration (ppm) | Hypha growing speed (µm/day) |
|---|---|---|---|---|
| Embodiment 4 | Blowing fan | Spray | 0.4 | 230 |
| Embodiment 5 | Air-blowoff blade | Spray | 0.5 | 250 |
| embodiment 6 | Blowing fan | Painting | 0.9 | 120 |
| Embodiment 7 | Air-blowoff blade | Painting | 0.8 | 160 |
| Comparative example | -- | -- | -- | 420 |

### Embodiments 8 to 10

Using an air conditioner having the same structure as that' shown in Fig.1, the mold-growth inhibiting effect by the heating process was checked.

The mold sensor as that used in the embodiments 1 to 7 was provided on a wall of a back surface of the blowing fan, an interior of the indoor unit was heated for a predetermined time at heating temperature and frequency as shown in Table 3, and growing speed of mold hypha of Alternaria sp. was measured. The interior of the indoor unit was heated by switching a refrigeration cycle of the air conditioner to so-called "heating operation" so that the heat exchanger functions as a condenser.

A thermocouple was disposed in the vicinity of the blowing fan to measure the heating temperature.

The heating process was carried out for two weeks under heating conditions shown in Table 3, and the growing speed of hypha after two weeks was measured.

The obtained mold growing speed and the like are shown in Table 3. A comparative example 1 is shown so that the results can be compared and considered.

**Table 3**

| | Heating temperature (°C) | Heating time (minutes) | Heating frequency | Hypha growing speed (µm/day) |
|---|---|---|---|---|
| Embodiment 8 | 40 | 10 | Once a day | 0 |
| Embodiment 9 | 50 | 10 | Once a day | 0 |
| Embodiment 10 | 60 | 10 | Once a day | 0 |
| Comparative example 1 | -- | -- | -- | 420 |
| Comparative example 5 | 40 | 10 | once a week | 420 |
| Comparative example 6 | 40 | 5 | Once a day | 430 |
| Comparative example 7 | 40 | 10 | Once for two days | 410 |
| Comparative example 8 | 35 | 10 | Once a day | 430 |
| Comparative example 9 | 35 | 20 | Once a day | 450 |

In embodiments 8 to 10, since the heating process was carried out by the heating operation, the heat exchanger was heated and its temperature increased and moisture condensation on its surface could be evaporated and removed, and surfaces of the heat exchanger, blower and air-blowoff blade could be heated to 36 °C or higher. Since the heating process at 36 °C or higher for 10 minutes or longer was carried out at least once a day, excellent hypha growth inhibiting effect could be obtained in any of the embodiments. The mold sensor used in the embodiment 8 was left in atmosphere of 30 °C and 95 %RH and as a result, the growing speed of hypha becomes 410 µm/day, and mold itself did not destroy oneself.

### Comparative Example 5

The heating frequency was set to once in a week, and other conditions are the same as those in the embodiment 8, and the heating process was carried out.

The obtained mold growing speed and the like are shown in Table 3.

### Comparative Example 6

The heating time was set to five minutes shorter than the range of the present invention. Other conditions are the same as those in the embodiment 8, and the heating process was carried out.

The obtained mold growing speed and the like are shown in Table 3.

### Comparative Example 7

The heating frequency was set to once for two days less than the range of the present invention. Other conditions are the same as those in the embodiment 8, and the heating process was carried out.

The obtained mold growing speed and the like are shown in Table 3.

### Comparative Examples 8 and 9

The heating temperature was set to 35 °C lower than the range of the present invention. Other conditions are the same as those in the embodiment 8, and the heating process was carried out.

The obtained mold growing speed and the like are shown in Table 3.

In the comparative examples 5 to 7, any of the conditions of heating time and frequency are out of the ranges of the present invention and thus, substantially the same results as that of the comparative example 1 which did not carry out the heating process were obtained. In the comparative examples 8 and 9, since the heating temperature was lower than the range of the invention, sufficient growing inhibiting effect could not be obtained. The reason why the growing speed of hypha was high although the heating process was carried out is due to variation in lots of mold sensor or measuring error of hypha. Since about 10% error may be caused, speeds of the comparative examples 1 and 5 to 9 are almost the same.

## Claims

1. An air conditioner including a body having an air blowing means, wherein said body is provided with a heating means so as to carry out heating at 36 °C or higher for 10 minutes or longer at least once a day, thereby inhibiting mold growth in said body.

2. The air conditioner according to claim 1, wherein at least part of the blowing means is heated.

3. The air conditioner according to claim 1 or 2, wherein said heating means is at least one of a heating equipment, a heater and a Peltier device for carrying out a normal heating operation.

4. An air conditioner including a body having an air blowing means, wherein said body is provided with a supplying means for supplying gas containing isothiocyanate, and a component constituting said blowing means is exposed at least once a day for 10 minutes or longer each to an atmosphere in which a concentration of isothiocyanate in a vapor phase is 0.1 ppm or higher.

5. An air conditioner including a body having an air blowing means, wherein said body is provided with a mold-preventing means in which isothiocyanate and resin are mixed with each other so as to lower a vapor pressure, and a concentration of isothiocyanate in a vapor phase in said body is set to 0.1 ppm or higher.

6. The air conditioner according to claim 5, wherein said resin includes at least one of urethane resin, acrylic resin and rosin ester resin.

7. An air conditioner including a body having an air blowing means, wherein at least one of components constituting said blowing means is applied with a paint containing isothiocyanate and having a gradually-releasing characteristics, so that a concentration of isothiocyanate in a vapor phase in said body is set to 0.1 ppm or higher.

8. The air conditioner according to claim 7, wherein said paint comprises a mixture of isothiocyanate and urethane resin or a mixture of isothiocyanate and acrylic resin.

9. The air conditioner according to claim 7, wherein said paint is prepared such that according as temperature rises, isothiocyanate is more easily discharged into the vapor phase.

10. An air conditioner including a body having an air blowing means, wherein said body is provided with a heating means and with a mold-preventing means in which isothiocyanate and resin are mixed with each other so as to lower a steam pressure, so that a concentration of isothiocyanate in a vapor phase in said body is set to 0.1 ppm or higher by heating with said heating means.

11. An air conditioner including a body having an air blowing means, wherein said body is provided with a heating means, at least one component constituting said blowing means is applied with a paint containing isothiocyanate and having a gradually-releasing characteristic, and the isothiocyanate applied to the component is evaporated by heating with said heating means, whereby a concentration of isothiocyanate in a vapor phase in said body is set to 0.1 ppm or higher.

12. A mold growth inhibiting method for an air conditioner including a body having a blowing means, wherein a gas containing isothiocyanate having a concentration of 0.1 ppm or higher is brought into contact, at least once a day, with a surface of a component constituting said blowing means, thereby inhibiting growth of mold.

13. A mold growth inhibiting method, in which a paint containing isothiocyanate and a gradually-releasing characteristic is applied to at least one component constituting a blowing means provided in a body of an air conditioner, and a concentration of isothiocyanate in a vapor phase in said body is set to 0.1 ppm or higher, thereby inhibiting growth of mold.

14. The mold growth inhibiting method according to claim 13, wherein said paint is prepared such that according as temperature rises, a discharge amount of isothiocyanate increases.

15. The mold growth inhibiting method according to claim 13 or 14, wherein the paint containing isothiocyanate and haing a gradually-releasing characteristic is applied or sprayed onto a surface of the component.

16. A mold growth inhibiting method, in which a body of an air conditioner is heated at 40 °C for 10 minutes at least once a day, thereby inhibiting growth of mold in said body.

17. The mold growth inhibiting method according to claim 16, wherein the body is heated by at least either a normal heating operation, or a heater or Peltier device provided in the body.

18. The mold growth inhibiting method according to claim 16 or 17, wherein the body is heated in a condition in which an air-blowoff port constituting a blowing means is closed.

19. The mold growth inhibiting method according to claim 16 or 17, wherein the body is heated in a condition in which an amount of blowing air by a blower constituting the blowing means is reduced as compared with that for a cooling operation.

20. The air conditioner according to claim 1 or 2, further comprising a control means for executing a heat treatment, at a set operation start time, by a normal heating operation for a predetermined time at a predetermined temperature, under an instruction to operate a mold-preventing heat treatment.

21. The air conditioner according to claim 20, wherein the control means is returned to a former cooling operation when the heat treatment has been completed.

22. The air conditioner according to claim 20, wherein the control means is provided to an input setting device so that said control means is capable of executing the heat treatment utilizing a timer provided to said input setting device.

23. The air conditioner according to claim 20, wherein when the mold-preventing operation start time is reached, the control means stops a cooling operation and switches to a mold-preventing operation, and the control means charges a gas containing isothiocyanate into an indoor unit.
